# EUROPEAN PATENT APPLICATION

(11) **EP 1 050 310 A1**
(43) Date of publication of application: **08.11.2000**
(21) Application number: 98957141.9
(22) Date of filing: 02.12.1998
(51) Int. Cl.: A61K 47/20

(54) **MINOXIDIL-CONTAINING LIQUID PREPARATION COMPOSITION**

(30) Priority: 03.12.1997 JP 33306097
(71) Applicant: TAISHO PHARMACEUTICAL CO., LTD, Tokyo 170-8633 (JP)
(72) Inventor: MIYAZAWA, Tomoki, Taisho Pharmaceutical Co., Ltd., Tokyo 170-8633 (JP)
(74) Representative: HOFFMANN - EITLE
(86) International application number: JP9805446
(87) International publication number: WO9927966

(57) **Abstract**

The minoxidil-containing liquid preparation tents to color with time, thereby it results in lowering the merchandise value. However, the liquid preparation containing minoxidil and an amino acid can be prevented from coloring with time, therefore the present invention makes it possible to give a commercially excellent liquid composition.

## Description

### TECHNICAL FIELD

The present invention relates to a minoxidil-containing liquid composition which is prevented from coloring with time.

### BACKGROUND ART

Minoxidil is a component used as an external hair growth agent because of having hair growth effect.

However, minoxidil, when stored in a liquid state, has a drawback of a tendency to color with time, and thus it results in lowering the merchandise value. The coloring mechanism of the minoxidil-containing liquid preparation is not necessarily made clear, but is presumed to be due to light. Therefore, it is necessary to use shaded containers for commercialization of a minoxidil-containing liquid preparation. However, these containers are inconvenient for use, for example, in that the residual content in the containers is not clear.

Generally, the liquid preparation containing the component which tends to color is improved in a combination with an antioxidant or an antichelating agent, however, such a combination is insufficient in an effect of preventing the minoxidil-containing liquid preparation from coloring with time.

On the other hand, in order to avoid the remarkability of color when a preparation colors, it is thought that the preparation is allowed to color from the first. However, since the hair growth preparation is used by the application to the scalp, the preparation which is allowed to color from the first is unpleasant owing to soiling clothes or bedclothes.

### DISCLOSURE OF THE INVENTION

As a result of various researches in order to prevent a minoxidil-containing liquid preparation from coloring, the present inventor has found that a minoxidil-containing liquid preparation can be surprisingly prevented from coloring by a combination with an amino acid, particularly taurine, thereby the present invention has been accomplished.

That is, the present invention is directed to a liquid composition containing minoxidil and an amino acid.

According to the present invention, the amino acid refers to any amino acids which can be contained in external liquid preparations, and includes a salt thereof.

Among the amino acids in the present invention, in view of the prevention effect on the coloring, preferable are sulfur-containing amino acids (e.g. taurine, cysteine, cystine, methionine or homocysteine), and especially taurine.

In the liquid composition of the present invention, a preferable concentration of minoxidil is from 0.1 to 5 % by weight based on the liquid preparation. In the case of less than 0.1 % by weight, the hair growth effect of minoxidil is weak, and in the case of more than 5 % by weight, it is difficult to design the preparation.

According to the present invention, the more the amount of the amino acid is, the higher the prevention effect on coloring of the liquid preparation is expected, but a preferable amount of the amino acid is from 0.01 to 20 parts by weight per 1 part by weight of minoxidil.

A preferable preparation solvent in the liquid composition of the present invention is an aqueous organic solvent, because the amino acid can not be dissolved in an organic solvent only, while minoxidil can not be dissolved in water only. The water content is preferably from 10 to 50 V/V %. It is necessary for the organic solvent used herein to be able to homogeneously mix with water and to give no adverse effect on the skin when used as a skin external preparation. In this view, preferable examples of the organic solvent are an alcohol having 1 to 6 carbon atoms (e.g. ethanol, propanol, isopropanol, ethylene glycol, propylene glycol, 1,3-butylene glycol, glycerol, etc.), and particularly preferable solvents are one or more members selected from the group consisting of ethanol, propylene glycol and 1,3-butylene glycol.

According to the present invention, in view of the prevention of the coloring, the liquid preparation is preferably adjusted to pH 4 - 9, when diluted to 20-fold with purified water.

The present invention makes it possible for a storage container for the minoxidil-containing liquid composition to be chosen from any containers used for an ordinary liquid preparation, but preferable materials of the container are PET, glass, polyethylene, polypropylene, etc.

The liquid preparation of the present invention can be produced by an ordinary method for producing a liquid preparation, however, when the amino acid is combined, preferable is a method wherein a solution of the amino acid which is previously dissolved in water is mixed with an organic solvent, because it takes long time to dissolve the amino acid in an aqueous organic solvent later in view of the dissolving rate of the amino acid.

The liquid preparation of the present invention may contain any components which are generally used for external liquid preparations (e.g. solubilizers, thickeners, absorption accelerators, pH regulators, etc.).

According to the present invention, the prevention effect on coloring of the preparation is recognized by a combination with the amino acid, and it is further expected to exert the pharmaceutical effects which the amino acid originally possesses (anti-inflammatory action, lowering action of skin irritation, antipruritic action, etc.) at the same time.

### BEST MODE FOR CARRYING OUT THE INVENTION

The present invention is illustrated in more detail by the following example and experiments.

### Example 1

One gram of minoxidil, 0.04 g of taurine, 10 g of propylene glycol, 60 g of ethanol and purified water to give 100 ml of the total volume were dissolved with stirring, thereby there was obtained a liquid preparation.

### Comparative Example 1

Following the same procedure as in Example 1 using the same formulation except for taurine, there was obtained a comparative liquid preparation.

### Experiment 1

Liquid preparations obtained in Example 1 and Comparative Example 1 were put into each 3 transparent glass bottles, and irradiated at room temperature with a light of 3000 lux for 34 days. The absorbance at the wave length of 420 nm, 1 cm of cell length, at the beginning and end of the experiment was measured. Results are shown in Table 1.

**Table 1**

| | Example 1 | | | Comparative Example 1 | | |
|---|---|---|---|---|---|---|
| | Test drug 1 | Test drug 2 | Test drug 3 | Test drug 1 | Test drug 2 | Test drug 3 |
| At the beginning of experiment | 0.002 | 0.002 | 0.002 | 0.001 | 0.001 | 0.001 |
| At the end of experiment | 0.056 | 0.050 | 0.058 | 0.090 | 0.077 | 0.097 |

### Example 2

One gram of minoxidil, 0.1 g of taurine, 10 g of propylene glycol, 60 g of ethanol and purified water to give 100 ml of the total volume were dissolved with stirring, thereby there was obtained a liquid preparation.

### Experiment 2

Liquid preparations obtained in Example 2 and Comparative Example 1 were each placed in a colorless glass bottle, and allowed to stand at room temperature by a window to receive sunlight for 6 months. After 30 days and 6 months, 3 surveyors who did not know the contents of the liquid preparations judged the coloring macroscopically. Furthermore, the absorbance at the wave length of 420 nm, 1 cm of cell length, at the beginning and after 6 months of the experiment was measured. Results are shown in Table 2.

**Table 2**

| | Example 2 | | Comparative Example 1 | |
|---|---|---|---|---|
| | Color | Absorbance | Color | Absorbance |
| At the beginning | Colorless | 0.002 | Colorless | 0.002 |
| 30 days | Colorless | - | Pale yellow | - |
| 6 months | Pale yellow | 0.152 | Conc. yellow | 0.508 |

### INDUSTRIAL APPLICABILITY

The present invention makes it possible to prevent the minoxidil-containing liquid preparation from coloring, and thus it can be used as a commercially excellent hair growth preparation.

## Claims

1. A liquid composition which contains minoxidil and an amino acid.

2. The liquid composition according to Claim 1 wherein the amino acid is a sulfur-containing amino acid.

3. The liquid composition according to Claim 1 wherein the amino acid is taurine.

4. The liquid composition according to Claim 1 wherein the amount of the amino acid is from 0.01 to 20 parts by weight per 1 part by weight of minoxidil.

5. The liquid composition according to any one of Claims 1 to 4 wherein a solvent to be used is a mixture of water and an alcohol having 1 to 6 carbon atoms.

6. The liquid composition according to Claim 5 wherein the water content in the mixture of water and the alcohol having 1 to 6 carbon atoms is from 10 to 50 v/v %.

7. The liquid composition according to Claim 5 wherein the alcohol having 1 to 6 carbon atoms is one or more members selected from the group consisting of ethanol, propylene glycol and 1,3-butylene glycol.

8. The liquid composition according to any one of Claims 1 to 7 wherein the amount of minoxidil is from 0.1 to 5 % by weight based on the whole preparation.

9. An agent for preventing a minoxidil-containing liquid composition from coloring which contains an amino acid as an effective component.

10. A method for preventing a minoxidil-containing liquid composition from coloring, which comprises adding an amino acid.
